# EUROPÄISCHE PATENTANMELDUNG

(11) **EP 0 604 769 A2**
(43) Veröffentlichungstag der Anmeldung: **06.07.1994**
(21) Anmeldenummer: 93119111.8
(22) Anmeldetag: 26.11.1993
(51) Int. Cl.: A61L 9/01

(54) **Sanitärkonzentrat zum Zusatz in Fäkalien**

(30) Priorität: 27.11.1992 DE 9216125 U
(71) Anmelder: Pirotte Pirofex KG, 53773 Hennef (DE)
(72) Erfinder: Pirotte, Wilhelm, Hennef-Uckerath (DE)
(74) Vertreter: Riederer Freiherr von Paar zu Schönau, Anton

(57) **Zusammenfassung**

Sanitärkonzentrat zum Zusatz in Fäkalien, zur Verhinderung starker Geruchsbildung bei der Lagerung der Fäkalien in weitgehend abgeschlossenen Behältern über einen Zeitraum von mehreren Tagen, wobei das Sanitärkonzentrat die Entwicklung der hauptsächlich für die Geruchsbildung verantwortlichen Fäulnisbakterien hemmt, und/oder die geruchstragenden Gase bindet indem es eine Substanz mit chemisch gebundenem Sauerstoff enthält, der über den obengenannten Zeitraum verteilt abgegeben wird, und/oder ihm Yuccapalmen-Extrakt zugesetzt wird.

## Beschreibung

Die Erfindung bezieht sich auf ein Sanitärkonzentrat zum Zusatz in Fäkalien, zur Verhinderung starker Geruchsbildung bei der Lagerung der Fäkalien in weitgehend geschlossenen Behältern über einen Zeitraum von mehreren Tagen, wobei das Sanitärkonzentrat die Entwicklung der hauptsächlich für die Geruchsbildung verantwortlichen Fäulnisbakterien hemmt und/oder die geruchstragenden Gase bindet. Die geschlossenen Behälter für Fäkalien sind insbesondere Bestandteile mobiler Toiletten. Sie sind naturgemäß nicht hermetisch geschlossen, sondern haben eine Einfüllöffnung, durch die auch Gase entweichen können, sind aber im übrigen ohne Luftaustausch mit der freien Atmosphäre.

Mobile Toiletten gibt es in vielerlei Formen. Neben den gewerblichen Miettoiletten, die z.B. für jede Baustelle vorgeschrieben sind, und Eisenbahn-Toilettensammelbehältern gibt es kleinere Toiletteneinheiten im Bereich Camping und Boote. All diesen mobilen Toiletten ist eigen, daß die dort abgelagerten Fäkalien geruchsfrei gehalten werden müssen, ehe sie nach z.B. 1 bis 10 Tagen in Klärwerke entsorgt werden. Es sind verschiedene Substanzen bekannt, die als Sanitärkonzentrat zur Behandlung der Fäkalien in den mobilen Behältern, insbesondere bei mobilen Toiletten, dienen. Ziel dieser Sanitär konzentrate ist es, die sonst starke Geruchsbelästigung, die bei der zeitlich begrenzten Lagerung in den mobilen Behältern entsteht, weitgehend zu verhindern.

Bei der unerwünschten Geruchsbildung entfällt auf die Gase, die direkt aus den Fäkalien freigesetzt werden, nur ein geringer Anteil. Hauptsächlich entsteht die Geruchsbelästigung dadurch, daß die Fäkalien in einem weitestgehend abgeschlossenen und damit luftdichten Behälter gelagert werden und dort von Fäulnisbakterien unter Bildung übelriechender Gase zersetzt werden. Es ist bekannt, daß die Geruchsbildung vor allem auf die durch Fäulnisbakterien hervorgerufenen Stoffumwandlungen zurückzuführen ist. Fäulnisbakterien benötigen für ihre Entwicklung und Vermehrung keinen Sauerstoff, im Gegensatz zu Rottebakterien, die eine Zersetzung ohne Bildung übelriechender Gase bewerkstelligen könnten. Beispielsweise wird in der Landwirtschaft eine starke Geruchsbelästigung dadurch vermieden, daß größere Mengen von Fäkalien mit geeigneten Hilfsmitteln durchlüftet werden. Eine solche Durchlüftung bietet den Vorteil, daß sich die damit erzielten Bedingungen besser für die Entwicklung von Rottebakterien eignen, als für die Entwicklung von Fäulnisbakterien. Sie ist jedoch in vielen Fällen, insbesondere bei mobilen Toiletten, aufgrund der notwendigen Kompaktheit der entsprechenden Behälter technisch kaum zu realisieren.

Zur Verhinderung der Geruchsbildung bei geschlossenen Behältern werden Sanitärkonzentrate eingesetzt, die mit Hilfe von Biociden jegliches bakterielle Wachstum behindern. Die meisten enthalten hohe Mengen von Aldehyden, vor allem Formaldeyd, von quartären Ammoniumverbindungen und dergleichen. All diese Zusätze sind für die Anwender belastend. Die hiermit versetzten Fäkalien erfordern hohe Verdünnungsraten im Klärwerk, um die für Klärwerke problematische antibakterielle Wirkung auszuschalten. Die ist bei großen Klärwerken weniger problematisch. Bedacht werden jedoch vielfach kleinere Einheiten, z.B. durch Camping in Feriengebieten, für die der zeitweise Anfall größerer "Chemiefäkalien" bedrohlich ist und zum "Kippen" der bakteriellen Umsetzung führen kann. Es wird deshalb der generelle Verzicht auf biocide Zusätze in Sanitärkonzentraten gefordert, ja sogar erwogen, die Biocidfäkalien als Sondermüll zu deklarieren, die speziell und mit abnorm hohen Kosten entsorgt werden müssen.

Nach dem Stand der Technik kann also die Geruchsbelästigung entweder kaum vermieden werden oder die Verhinderung der Bildung von geruchsintensiven Gasen geht mit Nachteilen einher, wie hohen Kosten für die entsprechenden Zusatzstoffe oder hoher chemischer Belastung der Umwelt bei der Entleerung der Fäkalienbehälter.

Der Erfindung liegt die Aufgabe zugrunde, durch Vermeidung der genannten Nachteile gleichzeitig eine starke Geruchsbelästigung, hohe Kosten und eine Belastung der Umwelt durch chemische Zusatzstoffe zu vermeiden.

Diese Aufgabe wird dadurch gelöst, daß das Sanitärkonzentrat eine Substanz mit chemisch gebundenem Sauerstoff, der über einen Zeitraum von mehreren Tagen verteilt abgegeben wird, und/oder Yuccapalmen-Extrakt enthält.

Vorzugsweise handelt es sich bei der Substanz mit dem chemisch gebundenen Sauerstoff um eine Substanz, die mit Wasser unter Abgabe von Sauerstoff reagiert. Dabei ist es günstig, wenn das Sanitärkonzentrat vorwiegend aus einem Peroxid, insbesondere aus Calciumperoxid, oder einem Percarbonat, insbesondere Natriumpercarbonat, besteht. Diese Stoffe, von denen Natriumpercarbonat 2Na₂CO₃·3H₂O₂ bevorzugt wird, bieten den Vorteil, daß sie den Sauerstoff bei der Reaktion mit Wasser nur sehr langsam und damit über einen längeren Zeitraum abgegeben, und zwar unter den üblichen Bedingungen, bei denen mobile Toiletten verwendet werden, d. h. in einem Temperaturbereich von -10°C bis +40°C.

Fügt man das Sanitärkonzentrat, welches den Sauerstoff abgibt, den Fäkalien bei, kann über einen längeren Zeitraum in der Fäkalien eine höhere Sauerstoffkonzentration erzielt werden. Der vorhandene Sauerstoff begünstigt die Entwicklung der Rottebakterien, welche bei den ablaufenden Abbauprozessen kaum geruchsintensive Gase erzeugen. Da man auf diese Art und Weise die Entwicklung der Rottebakterien fördert, wird gleichzeitig die Entwicklung der Fäulnisbakterien, die für den Hauptanteil der geruchsintensiven Gase verantwortlich sind, stark eingeschränkt. Das liegt daran, daß die verschiedenen Bakterienarten in unmittelbarer Nahrungskonkurrenz stehen.

Entstehende geruchstragende Gase, die gegebenenfalls trotz der Förderung der Rottebakterien auftreten, insbesondere Ammoniak NH₄, werden gemäß einem weiteren Aspekt der Erfindung durch Yuccapalmen-Extrakt gebunden. Die Yucca-Schidigera-Pflanze wächst in den Wüsten im Südwesten der Vereinigten Staaten und in der Baja-California-Region Mexikos. Mit einer Höhe von ca. 3 bis 4 m und einer Wachstumsdauer von 4 bis 5 Jahren prägt sie das Landschaftsbild der Wüste. Extrakte und Präparate der Yucca-Schidigera-Pflanze werden als Aromastoffe oder Schaummittel zur Herstellung alkohlfreier Getränke, in der Kosmetikindustrie wegen ihrer oberflächenaktiven Eigenschaften sowie in de Futtermittelindustrie eingesetzt.

Yucca-Extrakt ist im Handel unter der Handelsbezeichnung De-Odorase flüssig oder in kristalliner Form erhältlich. Dieses Yucca-Extrakt hat die Eigenschaft, gasbindend zu wirken, insbesondere für Ammoniak. Untersuchungen haben ergeben, daß hierfür nicht die darin enthaltenen, für die bekannten industriellen Anwendungen ausgenützten Saponine verantwortlich sind. Eine Beimengung des Extrakts beispielsweise in einer Menge von 2 Gewichtsprozent unterstützt die Geruchsunterdrückung nachhaltig. Das Sanitärkonzentrat kann dann beispielsweise aus 40 % Natriumcarbonat, 2 % Yuccapalmen-Extrakt und 50 % Trägerstoff bestehen. Sofern das Yuccapalmen-Extrakt ohne zusätzliche Stoffe als Sanitärkonzentrat verwendet wird, genügen beispielsweise Mengen zwischen 0,5 g und 0,75 g, um einen Toiletten-Sammelbehälter von 10 l Inhalt für einige Tage geruchsfrei zu halten.

Während bekannte Sanitärkonzentrate zumeist flüssig zur Verfügung stehen, wird im Rahmen der Erfindung eine Tabletten- oder Granulatform bevorzugt. Die Tablettenform hat den Vorteil, daß die Dosierung wesentlich einfacher zu handhaben ist. Auch die Granulatform kann einfach dosierbar sein, indem die vorgebene Menge in einer wasserlöslichen Folie gefaßt ist. Wenn auf die kristallinen Sauerstoffspender verzichtet wird, kann das Sanitärkonzentrat aber auch in flüssiger Form zur Anwendung kommen. Das Konzentrat kann beispielsweise so dosiert sein, daß eine Tablette für einen 10l-Sammelbehälter für eine Zeit von 5 Tagen genügt. Bei in Wohnmobilen vorkommenden mobilen Toiletten ist es üblich, dem Auffangbehälter für Fäkalien vor der ersten Benutzung eine bestimmte Menge Wasser zuzusetzen. Üblicherweise füllt man für je 10l Fassungsvermögen des Behälters etwa 1l Wasser ein. Diesem Wasser wird nun das Sanitärkonzentrat in Tablettenform zugesetzt. Im beschriebenen Beispiel enthält das Konzentrat einen Sauerstoffanteil von 10 bis 20%. Bei dieser Konzentration von Sauerstoff genügt es, je 10l Fäkalien ca. 25g des Sanitärkonzentrats zuzusetzen. Sobald das in Tablettenform vorliegende Konzentrat dem vor der ersten Benutzung der mobilen Toilette eingelassenen Wasser zugesetzt wird, beginnt eine chemische Reaktion abzulaufen. Bei dieser chemischen Reaktion reagiert die sauerstoffabgebende Substanz mit Wasser unter Freisetzung von Sauerstoff. Somit ist das Wasser mit Sauerstoff angereichert, der dann den in dem in das Wasser eingeleiteten Fäkalien vorhandenen Rottebakterien für ihre Stoffwechselprozesse zur Verfügung steht. Als zweites Reaktionsprodukt bleibt, bei den bevorzugten Substanzen, Calciumhydroxid bzw. Natriumcarbonat zurück. Diese Stoffe stellen bei der Entsorgung der mobilen Toiletten keine zusätzliche Umweltbelastung dar.

Die für das Sanitärkonzentrat verwendeten chemischen Stoffe werden so ausgewählt, daß sich Reaktionszeiten ergeben, die eine Freisetzung des Sauerstoffs über einen Zeitraum von mehreren Tagen zur Folge haben. Neben der Selbstregulierung dieser Abgabezeiten aufgrund der Reaktionszeiten läßt sich die Abgabezeit durch den Zusatz von Mitteln beeinflussen, die die Benetzbarkeit des Sanitärkonzentrats verändern. Z. B. können das Sprengmittel wie Bentonit sein. Dadurch wird die Benetzbarkeit der Sanitärkonzentrattablette erhöht und die Abgabe des Sauerstoffs beschleunigt.

Die Verwendung von Calciumperoxid oder Natriumpercarbonat als chemisch relevante Substanz für das Sanitärkonzentrat bietet weiterhin den Vorteil, daß das Konzentrat aufgrund seiner Ungiftigkeit einfach zu handhaben ist.

In dem Konzentrat können neben den chemischen Substanzen, aus denen der Sauerstoff freigesetzt wird, noch weitere Zusatzstoffe enthalten sein. So können Enzyme die gewünschte Verlaufsrichtung der Zersetzungsvorgänge zusätzlich steuern. Spontane Geruchsemissionen aus den Fäkalien werden durch den Zusatz von parfümierend und/oder desodorierend wirkenden Stoffen übertönt. Des weiteren ist der Zusatz von Farbstoffen denkbar, wodurch eine höhere optische Akzeptanz seitens des Anwenders erreichbar ist.

## Patentansprüche

1. Sanitärkonzentrat zum Zusatz in Fäkalien, zur Verhinderung starker Geruchsbildung bei der Lagerung der Fäkalien in weitgehend abgeschlossenen Behältern über einen Zeitraum von mehreren Tagen, wobei das Sanitärkonzentrat die Entwicklung der hauptsächlich für die Geruchsbildung verantwortlichen Fäulnisbakterien hemmt, dadurch gekennzeichnet, daß es eine Substanz mit chemisch gebundenem Sauerstoff enthält, der über den obengenannten Zeitraum verteilt abgegeben wird.

2. Sanitärkonzentrat zum Zusatz in Fäkalien, zur Verhinderung starker Geruchsbildung bei der Lagerung der Fäkalien in weitgehend abgeschlossenen Behältern über einen Zeitraum von mehreren Tagen, insbesondere nach Anspruch 1, dadurch gekennzeichnet, daß ihm Yuccapalmen-Extrakt zugesetzt ist.

3. Sanitärkonzentrat nach Anspruch 1 oder 2, dadurch gekennzeichnet, daß die Substanz mit dem chemisch gebundenen Sauerstoff eine Substanz ist, die mit Wasser unter Abgabe von Sauerstoff reagiert.

4. Sanitärkonzentrat nach einem der Ansprüche 1 bis 3, dadurch gekennzeichnet, daß die Substanz mit dem chemisch gebundenen Sauerstoff vorwiegend aus einem Peroxid, insbesondere aus Calciumperoxid besteht.

5. Sanitärkonzentrat nach einem der Ansprüche 1 bis 3, dadurch gekennzeichnet, daß die Substanz mit dem chemisch gebundenen Sauerstoff vorwiegend aus einem Percarbonat, insbesondere Natriumpercarbonat besteht.

6. Sanitärkonzentrat nach einem der Ansprüche 1 bis 5, dadurch gekennzeichnet, daß es 10% bis 20% Sauerstoff enthält.

7. Sanitärkonzentrat nach einem der Ansprüche 1 bis 6, dadurch gekennzeichnet, daß es des weiteren Zusatzstoffe mit parfümierender und/oder desodorierender Wirkung und/oder Farbstoffe enthält.

8. Sanitärkonzentrat nach einem der Ansprüche 1 bis 7, dadurch gekennzeichnet, daß es Mittel enthält, die seine Benetzbarkeit verändern.

9. Sanitärkonzentrat nach einem der Ansprüche 1 bis 8, dadurch gekennzeichnet, daß es als Feststoff in vordosierten Größen konfektioniert ist.

10. Sanitärkonzentrat nach dem auf Anspruch 2 rückbezogenen Anspruch 9, dadurch gekennzeichnet, daß eine Tablette für 10l Behälterinhalt zwischen 0,5 und 0,75 g Yuccapalmen-Extrakt enthält.
